# FASCICULE DE BREVET EUROPEEN

(11) **EP 1 155 687 B1**
(45) Date de publication et mention de la délivrance du brevet: **15.09.2004**
(21) Numéro de dépôt: 01401161.3
(22) Date de dépôt: 04.05.2001
(51) Int. Cl.: A61K 7/48, A61K 7/025

(54) **Procédé pour accroître la persistance d'au moins un effet cosmétique et/ou de soin d'une composition cosmétique, composition cosmétique et son utilisation**
Verfahren zur Erhöhung der Beständigkeit von mindestens eines pflege- oder kosmetischen Effekts einer kosmetischen Zusammenfassung , kosmetische Zusammensetzung und ihre Anwendung
Process to enhance the persistence of at least a cosmetic or care effect of a cosmetic composition, cosmetic composition and its use

(30) Priorité: 09.05.2000 FR 0005878
(43) Date de publication de la demande: 21.11.2001
(73) Titulaire: L'OREAL, 75008 Paris (FR)
(72) Inventeur: Mondet, Jean, 93600 Aulnay Sous Bois (FR); Mougin, Nathalie, 75011 Paris (FR)
(74) Mandataire: Dossmann, Gérard

(56) Documents cités:
- EP-A- 0 445 700
- EP-A- 0 706 790
- EP-A- 0 850 644
- EP-A- 0 979 643
- DE-A- 19 707 970
- FR-A- 2 765 800
- FR-A- 2 773 485
- US-A- 4 744 978
- US-A- 5 160 732
- US-A- 5 280 019
- ABED S ET AL: "SUPRAMOLECULAR ASSOCIATION OF ACID TERMINATED POLYDIMETHYLSIOXANES.1. SYNTHESIS AND CHARACTERIZATION" POLYMER BULLETIN,DE,SPRINGER VERLAG. HEIDELBERG, vol. 39, no. 3, 1 septembre 1997 (1997-09-01), pages 317-324, XP000721177 ISSN: 0170-0839

## Description

La présente invention concerne un procédé pour accroître la persistance d'au moins un effet cosmétique et/ou de soin d'une composition cosmétique, et une composition cosmétique, en particulier pour le maquillage et/ou le soin de la peau, y compris celle des paupières, ainsi que des lèvres et des phanères comme les cils, les sourcils, les ongles et les cheveux. Elle concerne également l'utilisation de cette composition cosmétique en tant que, par exemple, rouge à lèvres, mascara, eye-liner, fond de teint, poudre, fard à joues, ombre à paupières, maquillage de corps, produit hydratant, déodorant ou anti-transpirant.

En cosmétique, on cherche à obtenir des compositions qui, appliquées sur la peau, les lèvres et les phanères, forment des dépôts, par exemple, filmogènes, ayant un ou plusieurs effets cosmétiques et/ou de soin particuliers souhaités.

Ainsi, pour les compositions de maquillage telles que des rouges à lèvres, des mascaras et des "eye-liners", on cherche à obtenir des compositions qui forment des dépôts ayant des effets de coloration et/ou de brillance appropriés. Pour les compositions incluant un actif de soin telles que les produits hydratants, les déodorants et les anti-transpirants, on cherche en particulier à obtenir l'effet optimal de l'actif de soin présent dans la composition.

Dans tous les cas, on cherche également à obtenir la durée la plus longue du ou des effets cosmétiques et/ou de soin. Par exemple, pour les rouges à lèvres, les mascaras et les eye-liners, il est important d'obtenir une tenue prolongée de la coloration et/ou de la brillance ; pour les fonds de teint, les poudres, les fards à joues, les ombres à paupières et les maquillages de corps, il est important d'obtenir un effet mat persistant et durable malgré les frottements ou la sécrétion de sébum ou de sueur ; et pour les compositions incluant un actif de soin, une activité la plus longue possible de l'actif.

On a proposé d'incorporer des huiles de silicone dans des compositions cosmétiques. L'incorporation de ces huiles de silicone dans les compositions apporte aux dépôts des propriétés d'hydrophobicité, de brillance et de toucher non gras, mais les dépôts obtenus résistent mal aux agents externes comme la sueur ou le sébum, et en particulier aux agressions mécaniques comme le frottement.

Des polysiloxanes comportant des motifs amides et pouvant comporter des groupes capables d'établir des liaisons hydrogène, utilisés comme gélifiants des huiles de silicone de compositions cosmétiques, sont décrits dans le brevet WO 99/06473 de Colgate-Palmolive. On obtient ainsi des compositions généralement solides, transparentes ou translucides.

Le brevet US N° 5 919 441 de Colgate-Palmolive décrit une composition cosmétique à base d'un composant fluide comprenant au moins une silicone volatile ou non, et d'au moins un agent de gélification. Cet agent de gélification est un polymère contenant à la fois des motifs organosiloxy et des groupes formant des liaisons hydrogène qui sont choisis parmi les groupes ester, uréthanne, urée, thiourée, amide et leurs combinaisons. L'emploi d'un tel agent de gélification conduit, en particulier, à des compositions solides, de préférence transparentes ou translucides.

Le brevet FR 2 708 272 de Rhône-Poulenc décrit l'emploi comme adhésifs de polyorganosiloxanes comportant des groupes capables d'établir des liaisons hydrogène.

Selon l'invention, on a trouvé qu'il est possible d'accroître la persistance d'au moins un effet cosmétique et/ou de soin d'une composition cosmétique comprenant une phase huileuse, en incorporant à la phase huileuse, une quantité efficace d'au moins un organopolysiloxane, linéaire ou cyclique, comportant au moins deux motifs organosiloxy et au moins deux groupes latéraux et/ou terminaux capables d'établir chacun au moins une liaison hydrogène avec un ou des groupes partenaires.

Les organopolysiloxanes convenant dans l'invention comprennent au moins deux motifs organosiloxy qui peuvent être notamment représentés par la formule suivante :

RₐR'_{b}SiO_{(4-a-b)/2} (I)

dans laquelle :
R représente un groupe alkyle linéaire, ramifié ou cyclique, un groupe aryle, un groupe polyéther ou un groupe fluoré,
R' représente un groupe capable d'établir au moins une liaison hydrogène, de préférence au moins deux liaisons hydrogène,
a vaut 1, 2 ou 3, et
b vaut 0 ou 1, à condition que a+b soit égal à 2 ou 3.

Le nombre desdits motifs organosiloxy va de préférence de 2 à 50 000, et mieux encore de 2 à 30 000.

Les groupes alkyle peuvent être linéaires, ramifiés ou cycliques, et choisis notamment parmi les groupes méthyle, éthyle, propyle, isopropyle, n-butyle, sec-butyle, tert-butyle, pentyle; cyclopentyle, cyclohexyle et autres analogues. Le groupe méthyle est particulièrement préféré.

Parmi les groupes aryle, on préfère le groupe phényle.

Comme exemples de groupe polyéther, on peut citer les groupes polyoxyéthylène, polyoxypropyléne et polyoxyéthylène/polyoxypropylène.

Les groupes fluorés peuvent être des groupes alkyle linéaire, ramifié ou cyclique, ou alcényle, qui portent un ou plusieurs atomes de fluor comme substituants.

Les groupes R' sont des groupes latéraux et/ou terminaux capables d'établir des liaisons hydrogène et ils sont choisis de préférence parmi :
(a) les groupes dérivés d'acides aminés non protégés ou partiellement protégés, et
(b) les groupes acides carboxyliques, amines ou phénols de formule :

   -X-(Y)ₙ-Z

   dans laquelle :
   X représente une chaîne d'espacement linéaire, ramifiée ou cyclique, de type alkylène ou alcénylène, comportant éventuellement un ou plusieurs hétéroatomes dans la chaîne,
   Y représente un groupe hydrocarboné insaturé, divalent, monocyclique ou polycyclique, ou un groupe hétérocyclique insaturé divalent, ces groupes polycycliques ou hétérocycliques pouvant comporter jusqu'à 4 cycles condensés,
   n représente un nombre entier allant de 1 à 4, et
   Z représente un groupe -COOH, -OH ou un groupe amine primaire, secondaire ou tertiaire, dont l'atome d'azote fait éventuellement partie d'un groupe hétérocyclique Y.

On entend par persistance accrue d'au moins un effet cosmétique et/ou de soin d'une composition cosmétique selon l'invention, le fait qu'au moins un effet cosmétique et/ou de soin de la composition (par exemple, la brillance et/ou la coloration pour un rouge à lèvres, un mascara ou un eye-liner, la matité et la tenue de la couleur pour un fond de teint, une poudre ou un maquillage de corps, la suppression des odeurs corporelles pour un déodorant ou l'hydratation pour un produit hydratant) se maintient à son niveau initial ou près de son niveau initial pendant une durée significativement prolongée par comparaison avec une composition similaire mais ne comprenant pas d'organopolysiloxane ayant au moins deux groupes latéraux et/ou terminaux capables d'établir chacun au moins une liaison hydrogène, en particulier lors d'une exposition à des agents externes, et plus particulièrement lors d'une agression mécanique telle que le frottement.

Par quantité efficace d'un organopolysiloxane selon l'invention, on entend une quantité suffisante pour accroître de façon significative la persistance d'un ou de plusieurs effets cosmétiques et/ou de soin de la composition cosmétique.

Par groupe partenaire, on entend tout groupe latéral et/ou terminal porté par une autre molécule dudit organopolysiloxane, capable de former au moins une liaison hydrogène avec le groupe latéral et/ou terminal dudit organopolysiloxane. Ce groupe partenaire est identique ou non au groupe latéral et/ou terminal avec lequel il forme au moins une liaison hydrogène.

Par phase huileuse, on entend un milieu non aqueux liquide à température ambiante (25 °C) et sous pression atmosphérique (1,013 x 10⁵ Pa (760 mm Hg)), contenant un ou plusieurs corps gras liquides à température ambiante et sous pression atmosphérique, généralement miscibles entre eux.

La présente invention a donc pour objet un procédé pour accroître la persistance d'au moins un effet cosmétique et/ou de soin d'une composition cosmétique.

La présente invention a encore pour objet une composition cosmétique, en particulier pour le maquillage et/ou le soin de la peau, des lèvres et des phanères comme les cils, les sourcils, les ongles et les cheveux, ayant une persistance accrue d'au moins un effet cosmétique et/ou de soin.

Un autre objet de la présente invention est l'utilisation d'une composition cosmétique selon l'invention en tant que rouge à lèvres, mascara, eye-liner, fond de teint, poudre, fard à joues, ombre à paupières, maquillage de corps, produit hydratant, déodorant ou anti-transpirant. Tous ces produits présentent alors au moins un effet cosmétique et/ou de soin dont la persistance est accrue.

D'autres caractéristiques, aspects et avantages de l'invention apparaîtront encore plus clairement à la lecture de la description et des divers exemples qui suivent.

Un objet de l'invention concerne un procédé pour accroître la persistance d'au moins un effet cosmétique et/ou de soin d'une composition cosmétique comprenant une phase huileuse. Ce procédé comprend l'incorporation à ladite phase huileuse, d'une quantité efficace d'au moins un organopolysiloxane, linéaire ou cyclique, comportant au moins deux motifs organosiloxy et au moins deux groupes latéraux et/ou terminaux capables d'établir chacun au moins une liaison hydrogène avec un ou des groupes partenaires.

Les organopolysiloxanes convenant dans l'invention comprennent au moins deux motifs organosiloxy qui peuvent être notamment représentés par la formule suivante :

RₐR'_{b}SiO_{(4-a-b)/2} (I)

dans laquelle :
R représente un groupe alkyle linéaire, ramifié ou cyclique, un groupe aryle, un groupe polyéther ou un groupe fluoré,
R' représente un groupe capable d'établir au moins une liaison hydrogène, de préférence au moins deux liaisons hydrogène,
a vaut 1, 2 ou 3, et
b vaut 0 ou 1, à condition que a+b soit égal à 2 ou 3.

Le nombre desdits motifs organosiloxy va de préférence de 2 à 50 000, et mieux encore de 2 à 30 000.

Les groupes alkyle peuvent être linéaires, ramifiés ou cycliques, et choisis notamment parmi les groupes méthyle, éthyle, propyle, isopropyle, n-butyle, sec-butyle, tert-butyle, pentyle, cyclopentyle, cyclohexyle et autres analogues. Le groupe méthyle est particulièrement préféré.

Parmi les groupes aryle, on préfère le groupe phényle.

Comme exemples de groupe polyéther, on peut citer les groupes polyoxyéthylène, polyoxypropylène et polyoxyéthylène/polyoxypropylène.

Les groupes fluorés peuvent être des groupes alkyle linéaire, ramifié ou cyclique, ou alcényle, qui portent un ou plusieurs atomes de fluor comme substituants.

Les groupes R' sont des groupes latéraux et/ou terminaux capables d'établir des liaisons hydrogène et ils sont choisis de préférence parmi :
(a) les groupes dérivés d'acides aminés non protégés ou partiellement protégés, et
(b) les groupes acides carboxyliques, amines ou phénols de formule :

   -X-(Y)ₙ-Z

   dans laquelle :
   X représente une chaîne d'espacement linéaire, ramifiée ou cyclique, de type alkylène ou alcénylène, comportant éventuellement un ou plusieurs hétéroatomes dans la chaîne,
   Y représente un groupe hydrocarboné insaturé, divalent, monocyclique ou polycyclique, ou un groupe hétérocyclique insaturé divalent, ces groupes polycycliques ou hétérocycliques pouvant comporter jusqu'à 4 cycles condensés,
   n représente un nombre entier allant de 1 à 4, et
   Z représente un groupe -COOH, -OH ou un groupe amine primaire, secondaire ou tertiaire, dont l'atome d'azote fait éventuellement partie d'un groupe hétérocyclique Y.

Comme cela est bien connu dans la technique, un groupe acide carboxylique peut former des liaisons hydrogène avec un autre groupe acide carboxylique ou un groupe amine, alors qu'un groupe amine peut former des liaisons hydrogène avec un groupe acide carboxylique ou un groupe OH phénolique.

Ainsi, on peut utiliser dans le procédé de l'invention, un unique organopolysiloxane ayant au moins deux groupes terminaux et/ou latéraux dont au moins l'un est un groupe -COOH et au moins un autre est un groupe -COOH ou amine (primaire, secondaire ou tertiaire), ou bien un unique organopolysiloxane ayant au moins deux groupes terminaux et/ou latéraux dont au moins l'un est un groupe amine (primaire, secondaire ou tertiaire) et au moins un autre est un groupe -OH phénolique ou -COOH.

On peut également utiliser des mélanges, de préférence équimolaires, de deux organopolysiloxanes comportant des groupes partenaires. Ainsi, on peut utiliser un mélange d'un organopolysiloxane ayant au moins deux groupes -COOH avec un organopolysiloxane ayant au moins deux groupes amines, ou bien un mélange d'un organopolysiloxane ayant au moins deux groupes amines et d'un organopolysiloxane ayant au moins deux groupes -OH phénoliques.

Les fonctions amine et/ou acide carboxylique des groupes dérivés d'acides aminés peuvent être non protégées ou partiellement protégées par des groupes particuliers tels que le groupe acétyle. Comme exemples de groupes dérivés d'acides aminés, on peut citer la cystéine, la N-acétylcystéine, le glycocolle, l'alanine, la sérine, la N-acétylcystéine étant particulièrement préférée.

La fonctionnalisation de l'organopolysiloxane par ces groupes dérivés d'acides aminés se fait par des techniques bien connues de l'homme de métier, telles que la silylation des liaisons insaturées par un dérivé thiol de l'acide aminé ou par réaction d'un organohydrogénosiloxane avec un dérivé d'acide aminé porteur d'une liaison insaturée.

La chaîne d'espacement X est un groupe alkylène ou alcénylène, linéaire, ramifié ou cyclique, qui peut comporter un ou plusieurs hétéroatomes tels que N, S ou O. A titre d'exemple de chaîne d'espacement, on peut citer -(CH₂)ₚ-S- ou -(CH₂)ₚ-O-, p variant de préférence de 1 à 5.

Parmi les groupes hydrocarbonés insaturés, divalents, monocycliques ou polycycliques, ou hétérocycliques insaturés, Y représente de préférence un noyau aromatique à 6 chaînons, pouvant comporter un ou plusieurs hétéroatomes.

Comme groupe hydrocarboné insaturé, divalent, monocyclique ou polycyclique, on peut citer les groupes phénylène ou naphtalène-diyle, le groupe phénylène étant particulièrement préféré.

Dans le cas où Y représente un groupe hétérocyclique insaturé, contenant un atome d'azote par exemple, Z peut représenter un groupe amine dont l'atome d'azote fait partie d'un groupe hétérocyclique Y et Y-Z est notamment choisi parmi les groupes pyridyle, pyrimidinyle, diazanaphtalène-diyle.

Les organopolysiloxanes convenant dans la présente invention, sont les organopolysiloxanes, linéaires ou cycliques, comportant au moins deux motifs organosiloxy et au moins deux groupes latéraux et/ou terminaux capables d'établir des liaisons hydrogène, tels que ceux décrits dans le document FR 2 708 272 cité ci-dessus.

On utilise les organopolysiloxanes convenant dans l'invention en une quantité se situant généralement dans l'intervalle allant de 0,5 à 50 % en poids, de préférence de 1 à 30 % en poids par rapport au poids total de la composition cosmétique.

La phase huileuse que l'on peut utiliser dans le procédé de l'invention, est constituée de toute huile cosmétiquement acceptable, notamment choisie parmi les huiles d'origine minérale, animale, végétale ou synthétique, hydrocarbonées et/ou siliconées, seules ou en mélange dans la mesure où elles forment un mélange homogène et stable, et où elles sont compatibles avec l'utilisation envisagée. Cette phase huileuse comprend donc au moins une huile hydrocarbonée et/ou au moins une huile de silicone, de préférence au moins une huile de silicone volatile ou non.

Par milieu (ou huile) cosmétiquement acceptable, on entend un milieu (ou huile) compatible avec la peau, les lèvres et/ou les phanères mais aussi d'odeur, d'aspect et de toucher agréables.

Les huiles de silicone peuvent être choisies parmi les polydiméthylsiloxanes (PDMS), éventuellement phénylés tels que les phényltriméthicones, les phényltriméthylsiloxydiphénylsiloxanes, les diphénylméthyldiméthyltrisiloxanes, les diphényldiméthicones, les phényldiméthicones, les polyméthyl-phénylsiloxanes, éventuellement substitués par des groupements aliphatiques et/ou aromatiques, ou éventuellement fluorés ; les poly-siloxanes modifiés par des acides gras, des alcools gras ou des polyoxyalkylènes, les silicones fluorées, les huiles siliconées perfluorées et leurs mélanges.

Parmi les huiles de silicone préférées, on peut citer les polydiméthylsiloxanes, les polyméthylphénylsiloxanes, les silicones comportant des séquences ou des greffons polyoxyalkylènes, en particulier polyoxyéthylène ou copoly(oxyéthylène/oxypropylène) telles que les diméthiconecopolyols, les silicones portant à la fois des groupes hydrophobes hydrocarbonés (par exemple des groupes alkyle en C₂-C₃₀) et des séquences ou greffons polyoxyéthylénés ou copoly(oxyéthylénés/oxypropylénés) telles que les alkyldiméthiconecopolyols, les silicones portant des groupes fluorés ou perfluorés telles que les polydiméthylsiloxanes perfluoroalkylés et les polyméthylphénylsiloxanes perfluoroalkylés, et leurs mélanges.

On peut aussi utiliser de manière avantageuse une ou plusieurs huiles volatiles à température ambiante. Après évaporation de ces huiles, on obtient un dépôt filmogène souple. Ces huiles volatiles facilitent, en outre, l'application de la composition sur la peau, les lèvres et les phanères.

Par huile volatile, on entend une huile capable de s'évaporer à la température de la peau ou des lèvres, présentant une pression de vapeur non nulle à température ambiante et sous la pression atmosphérique, allant en particulier de 0,13 à 4,0x10⁴ Pa (10⁻³ à 300 mm Hg), et mieux encore supérieure à 40 Pa (0,3 mm Hg).

Ces huiles peuvent être des huiles de silicones comportant éventuellement des groupes alkyle ou alcoxy en bout de chaîne siliconée ou pendante.

Comme huile de silicone volatile utilisable dans l'invention, on peut citer les silicones linéaires ou cycliques, ayant une viscosité à température ambiante et sous pression atmosphérique inférieure à 8 mm²/s (8 cSt), et comportant en particulier de 2 à 7 atomes de silicium. On peut notamment citer l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane et leurs mélanges.

De préférence, on peut utiliser au moins une huile de silicone volatile choisie notamment parmi l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane, l'octaméthyltrisiloxane, le décaméthyltétrasiloxane et leurs mélanges.

Par huile hydrocarbonée, on entend une huile contenant majoritairement des atomes de carbone et d'hydrogène et notamment des chaînes alkyle ou alcényle comme les alcanes ou les alcènes, mais aussi une huile contenant en plus des atomes d'hydrogène et de carbone, des atomes d'oxygène sous forme de fonction éther, ester, alcool ou acide carboxylique.

On peut ainsi citer les huiles hydrocarbonées telles que l'huile de paraffine ou de vaseline, l'huile de vison, de tortue, de soja, le perhydrosqualène, l'huile d'amande douce, de calophyllum, de palme, de pépins de raisin, de sésame, de maïs, de parléam, d'arara, de colza, de tournesol, de coton, d'abricot, de ricin, d'avocat, de jojoba, d'olive ou de germes de céréales; des esters d'acide lanolique, d'acide oléique, d'acide laurique, d'acide stéarique; les esters gras, tels que le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle, le succinate de di(2-éthylhexyle), le malate de diisostéaryle, le triisostéarate de glycérine ou de diglycérine ; les alcools gras supérieurs à au moins 12 atomes de carbone tels que l'alcool stéarylique ou l'alcool oléique, l'alcool linoléique ou linolénique, l'alcool isostéarique ou l'octyldodécanol.

Dans un mode particulier de réalisation, le procédé permet d'accroître la persistance de la coloration et/ou de la brillance d'un rouge à lèvres par l'incorporation d'un organopolysiloxane tel que décrit ci-dessus dans ladite phase huileuse.

Le procédé selon l'invention permet également d'accroître la persistance de la matité et/ou la coloration des fonds de teint, des poudres, des fards à joues ou des ombres à paupières.

Dans un autre mode de réalisation, la persistance d'un effet de soin est obtenu avec le procédé de l'invention, en particulier dans le cas des produits hydratants, des déodorants et des anti-transpirants. Une gélification peut être observée comme dans les brevets WO 99/06473 et US N° 5 919 441 de Colgate-Palmolive qui utilisent des polysiloxanes différents de l'invention, et permet d'obtenir des produits utilisables, par exemple, en tant que déodorants.

Un autre objet de la présente invention est une composition cosmétique pour le maquillage et/ou le soin de la peau, des lèvres et des phanères, dont la persistance d'au moins un effet cosmétique et/ou de soin est accrue. Cette composition comprend une phase huileuse comportant au moins une huile de silicone, volatile ou non, dans laquelle on a incorporé au moins un organopolysiloxane approprié comportant au moins deux motifs organosiloxy et au moins deux groupes latéraux et/ou terminaux capables d'établir chacun au moins une, de préférence au moins deux liaisons hydrogène avec un ou des groupes partenaires.

Lesdits motifs organosiloxy sont représentés par à la formule (I) ci-dessus et lesdits groupes latéraux et/ou terminaux R' de la formule (I) répondent à la définition indiquée ci-dessus.

La préparation des organopolysiloxanes des compositions de l'invention est connue dans la technique. Des exemples de préparation sont décrits dans le brevet français numéro 2 708 272 de Rhône-Poulenc et dans l'article de S. Abed et al, Polym. Mater. Sci. Eng., 1997, N° 76, 45-46.

Un premier exemple de préparation consiste à utiliser un organopolysiloxane présentant des groupes latéraux insaturés, tels que des groupes vinyliques ou allyliques, et à le faire réagir avec un dérivé sulfanylique comme la N-acétylcystéine. On peut alors obtenir un organopolysiloxane comportant les motifs suivants :

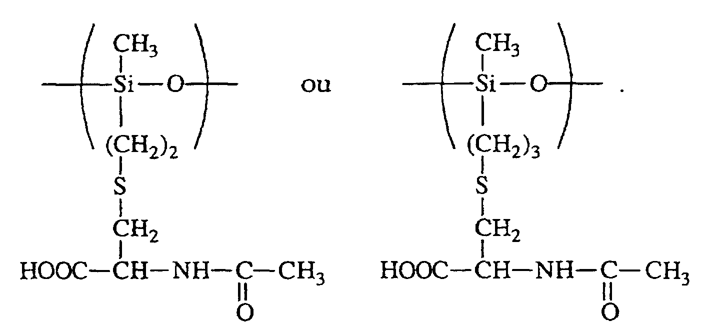

Un deuxième exemple de préparation consiste à utiliser un organopolysiloxane présentant des groupes silyle -SiH et à le faire réagir par hydrosilylation avec un dérivé d'acide aminé porteur d'une double liaison vinylique ou allylique, dont on a neutralisé par silylation les fonctions acide carboxylique et amine, comme par exemple : 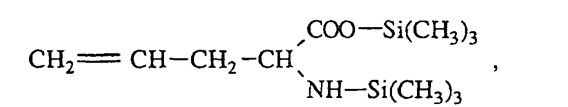 puis une fois la réaction d'hydrosilylation terminée, à déprotéger les fonctions acide carboxylique et amine. On obtient alors le même type de motifs que ceux représentés ci-dessus.

La synthèse d'organopolysiloxanes à groupes terminaux p-carboxyphényloxy est décrite dans l'article de S. Abed et al, Polym. Bull., 39, 1997, pages 317-324. Elle consiste à préparer tout d'abord un p-allyloxybenzoate de benzyle et à le faire réagir avec un organopolysiloxane à groupes terminaux -SiH par hydrosilylation. La dernière étape consiste à déprotéger les groupes terminaux pour obtenir finalement le produit suivant : 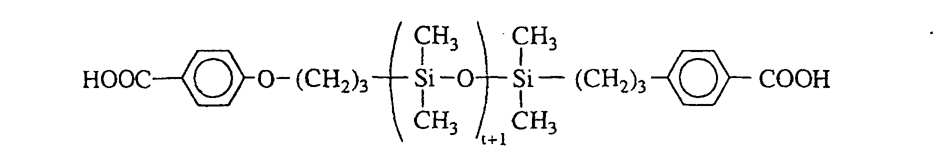 avec t allant de préférence de 1 à 1200.

Selon l'invention, on utilise de préférence l'organopolysiloxane répondant à la formule ci-dessus avec t=11.

La phase huileuse de la composition cosmétique selon l'invention comprend de préférence au moins une huile de silicone, volatile ou non, telle que décrite ci-dessus, et peut comprendre une huile hydrocarbonée telle que mentionnée ci-dessus.

La composition selon l'invention peut comprendre, en outre, au moins un ingrédient choisi parmi les actifs cosmétiques et/ou les actifs de soin selon le type d'application envisagée, ainsi que divers autres additifs classiques utilisés dans le domaine des cosmétiques, tels que, par exemple, des charges, des pigments, des colorants, des tensio-actifs, des filtres solaires, des cires naturelles ou synthétiques, des anti-oxydants, des parfums, des conservateurs.

Les actifs cosmétiques et/ou de soin sont utilisés en une proportion habituelle pour l'homme de métier, et notamment en une proportion allant de 0,001 à 30 % en poids de la composition.

L'homme de métier veillera à choisir les éventuels additifs et leur quantité de manière à ce qu'ils ne nuisent pas aux propriétés des compositions de l'invention, c'est-à-dire qu'ils ne doivent pas être capables de former des liaisons hydrogène avec les composants principaux de la composition cosmétique, qui ont été décrits ci-dessus.

Les charges peuvent être minérales ou organiques, lamellaires ou sphériques. On peut citer le talc, le mica, la silice, le kaolin, les poudres de Nylon® (Orgasol® d'Atochem), de poly-β-alanine et de polyéthylène, le Téflon®, la lauroyl-lysine, l'amidon, le nitrure de bore, les microsphères creuses telles que l'Expancel® (Nobel Industrie), le Polytrap® (Dow Corning) et les microbilles de résine de silicone (Tospearls® de Toshiba, par exemple), le carbonate de calcium précité, la carbonate et l'hydrocarbonate de magnésium, l'hydroxyapatite, les microsphères de silice creuses (Silica beads de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium.

Les pigments nacrés peuvent être choisis parmi les pigments nacrés blancs tels que le mica recouvert d'oxyde de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont, par exemple, le rouge Soudan, le DC Red 17, le DC Green 6, le β-carotène, le DC Yellow 11, le DC Violet 2. Ils peuvent représenter de 0,01 à 20 % du poids de la composition et mieux de 0,1 à 6 %.

Les tensio-actifs peuvent être des tensioactifs anioniques, cationiques ou non-ioniques.

Les filtres solaires sont choisis parmi les filtres solaires actifs dans l'UV-A et l'UV-B.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires pouvant être utilisées selon l'invention, on peut citer les cires d'origine animale telles que la cire d'abeilles, le spermaceti, la cire de lanoline et les dérivés de lanoline ; les cires végétales telles que la cire de Carnauba, de Candellila, d'Ouricury, du Japon, le beurre de cacao ou les cires de fibres de liège ou de canne à sucre ; les cires minérales, par exemple de paraffine, de vaseline, de lignite ou les cires microcristallines ou les ozokérites ; les cires synthétiques parmi lesquelles les cires de polyéthylène, de polytétrafluoroéthylène et les cires obtenues par synthèse de Fisher-Tropsch ou encore les cires de silicone, les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale "KESTER WAX K82H" par la société KOSTER KEUNEN.

Dans un mode de réalisation particulier de l'invention, les compositions selon l'invention peuvent être préparées de manière usuelle par l'homme du métier et peuvent se présenter sous forme d'un produit coulé et, par exemple, sous la forme d'un stick ou bâton, ou sous la forme de coupelle utilisable par contact direct ou d'une éponge. En particulier, elles trouvent une application en tant que fond de teint coulé, fard à joues ou à paupières coulé, rouge à lèvres, base ou baume de soin pour les lèvres, produit anti-cernes, déodorant et anti-transpirant, produit de maquillage du corps tel qu'un tatouage semi-permanent, produit solaire ou mascara pain. Elles peuvent aussi se présenter sous forme d'une pâte souple, de viscosité dynamique à 25°C de l'ordre de 1 à 40 Pa.s, telle que mesurée sur un appareil Haake RS 50, avec une viscosité extrapolée aux gradients de cisaillement inférieurs à 1 s⁻¹.

Les compositions de l'invention sont avantageusement anhydres et peuvent contenir jusqu'à 5% d'eau par rapport au poids total de la composition. Elles peuvent alors se présenter notamment sous forme de gel huileux, de liquide huileux ou huile, de pâte ou de stick. Ces différentes formes sont préparées selon les méthodes usuelles des domaines considérés.

Les compositions de l'invention peuvent être utilisées, par exemple, pour accroître la persistance de la brillance et/ou de la coloration d'un rouge à lèvres, d'un mascara ou d'un eye-liner pour accroître la persistance de la matité et/ou de la coloration des fonds de teint, des poudres, des fards à joues, des ombres à paupières ou des maquillages de corps comme des tatouages semi-permanents, pour accroître la persistance des effets de soin d'un produit hydratant ou pour supprimer durablement les odeurs corporelles dans le cas d'un déodorant ou d'un anti-transpirant.

Les exemples suivants illustrent la présente invention.

### Synthèse du composé A

Un polyorganosiloxane à groupes terminaux p-carboxyphényloxy tel que défini ci-dessus, est préparé selon le procédé décrit dans l'article de S. ABED, Polymer Bulletin, 39, 317-324 (1997). On obtient ainsi le composé A de formule suivante :

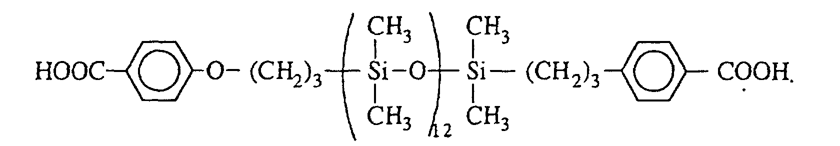

Ce composé A est utilisé dans les exemples suivants de préparation de rouge à lèvres et de brillant à lèvres.

### Exemple 1

On prépare un rouge à lèvres ayant la composition suivante :

| | |
|---|---|
| Cire de polyéthylène PERFORMALENE 500® ⁽¹⁾ | 15 g |
| Composé A | 5 g |
| Pigments | 9 g |
| Huile de polyisobutylène hydrogéné⁽²⁾ | 35,5 g |
| Huile de phényltriméthicone Dow 556 Fluid® ⁽³⁾ | 35,5 g |

| | |
|---|---|
| (1) vendue par la société PETROLITE | |
| (2) de viscosité 34 mm²/s (34 cSt) à 25 °C, vendue sous le nom de "Parléam® " par la société NIPPON OIL-FATS. | |
| (3) vendue par la société DOW CORNING. | |

On mélange à 110 °C tous les constituants de la composition ci-dessus. Après homogénéisation et broyage des pigments, on coule le mélange dans un moule adéquat. On obtient ainsi un stick présentant de bonnes caractéristiques rhéologiques. Il permet de déposer sur les lèvres un film présentant une bonne tenue dans le temps.

### Exemple 2

On prépare un brillant à lèvres ayant la composition suivante :

| | |
|---|---|
| Composé A | 5 g |
| Pigment (DC Red N° 7 Calcium (laque)) | 5 g |
| Huile de phényltriméthicone Dow 556 Fluid® ^{(*)} | 90 g |

| | |
|---|---|
| (*) vendue par la société DOW CORNING. | |

Le composé A est tout d'abord dissous dans l'huile. On obtient un brillant à lèvres en dispersant les pigments dans cette phase huileuse. Le brillant à lèvres ainsi obtenu peut être appliqué au pinceau sur les lèvres. Il confère une coloration brillante durable dans le temps.

## Revendications

1. Procédé pour accroître la persistance d'au moins un effet cosmétique et/ou de soin d'une composition cosmétique comprenant une phase huileuse, **caractérisé en ce qu'**il comprend l'addition à la composition d'une quantité efficace d'au moins un organopolysiloxane, linéaire ou cyclique, comportant au moins deux motifs organosiloxy et au moins deux groupes latéraux et/ou terminaux capables d'établir chacun au moins une liaison hydrogène avec un ou des groupes partenaires, lesdits motifs organosiloxy étant représentés par la formule suivante :
RₐR'_{b}SiO_{(4-a-b)/2}
dans laquelle :
R représente un groupe alkyle linéaire, ramifié ou cyclique, un groupe aryle, un groupe polyéther ou un groupe fluoré,
R' représente un groupe capable d'établir au moins une liaison hydrogène,
a vaut 1, 2 ou 3, et
b vaut 0 ou 1, à condition que a+b soit égal à 2 ou 3;
et lesdits groupes R' capables d'établir au moins une liaison hydrogène étant choisis parmi :
(a) les groupes dérivés d'acides aminés non protégés ou partiellement protégés, et
(b) les groupes acides carboxyliques, amines ou phénols de formule :
-X-(Y)ₙ-Z
dans laquelle :
X représente une chaîne d'espacement linéaire, ramifiée ou cyclique, de type alkylène ou alcénylène, comportant éventuellement un ou plusieurs hétéroatomes dans la chaîne ;
Y représente un groupe hydrocarboné insaturé, divalent, monocyclique ou polycyclique, ou un groupe hétérocyclique insaturé divalent, ces groupes polycycliques ou hétérocycliques pouvant comporter jusqu'à 4 cycles condensés,
n représente un nombre entier allant de 1 à 4,
Z représente un groupe -COOH, -OH, ou un groupe amine primaire, secondaire ou tertiaire, dont l'atome d'azote fait éventuellement partie d'un groupe hétérocyclique Y.

2. Procédé selon la revendication 1, **caractérisé en ce que** l'organopolysiloxane comporte de 2 à 50 000 motifs organosiloxy, de préférence de 2 à 30 000 motifs organosiloxy.

3. Procédé selon selon la revendication 1 ou 2, **caractérisé en ce que** les groupes latéraux et/ou terminaux sont capables d'établir chacun au moins deux liaisons hydrogène avec un ou des groupes partenaires.

4. Procédé selon l'une quelconque des revendications précédentes, **caractérisé en ce que** Y représente un noyau aromatique à 6 chaînons et Z représente un groupe -COOH.

5. Procédé selon l'une quelconque des revendications précédentes, **caractérisé en ce que** l'organopolysiloxane est représenté par la formule suivante : 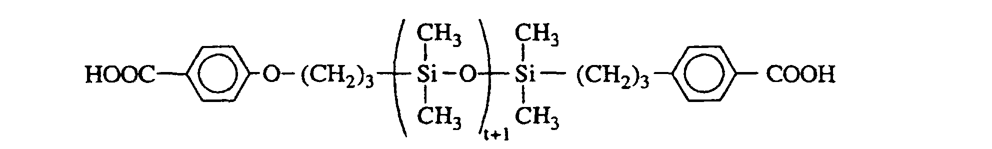 avec t allant de préférence de 1 à 1200.

6. Procédé selon la revendication 5, **caractérisé en ce que** l'organopolysiloxane est représenté par la formule suivante :

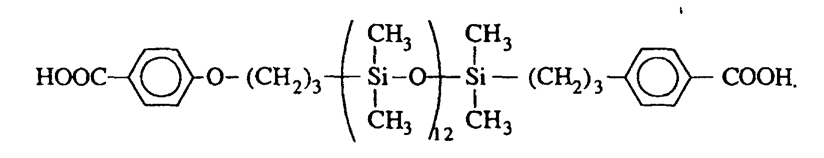

7. Procédé selon l'une quelconque des revendications 1 à 3, **caractérisé en ce que** lorsque Z représente un groupe amine, l'atome d'azote fait partie d'un groupe hétérocyclique Y, et Y-Z représente un groupe pyridyle.

8. Procédé selon l'une quelconque des revendications précédentes, **caractérisé en ce que** la quantité efficace de l'organopolysiloxane est comprise entre 0,5 et 50 % en poids, par rapport au poids total de la composition cosmétique.

9. Procédé selon la revendication 8, **caractérisé en ce que** la quantité efficace de l'organopolysiloxane est comprise entre 1 et 30 % en poids, par rapport au poids total de la composition cosmétique.

10. Procédé selon l'une quelconque des revendications précédentes, **caractérisé en ce que** la phase huileuse comprend au moins une huile hydrocarbonée et/ou au moins une huile de silicone.

11. Procédé selon la revendication 10, **caractérisé en ce que** la phase huileuse contient au moins une huile de silicone volatile ou non.

12. Procédé selon la revendication 10 ou 11, **caractérisé en ce que** l'huile de silicone est choisie parmi les polydiméthylsiloxanes (PDMS), éventuellement phénylés tels que les phényltriméthicones, les phényltriméthylsiloxydiphénylsiloxanes, les diphénylméthyldiméthyltrisiloxanes, les diphényldiméthicones, les phényldiméthicones, les polyméthylphénylsiloxanes, éventuellement substitués par des groupements aliphatiques et/ou aromatiques, ou éventuellement fluorés ; les polysiloxanes modifiés par des acides gras, des alcools gras ou des polyoxyalkylènes, les silicones fluorées et les huiles siliconées perfluorées.

13. Procédé selon la revendication 12, **caractérisé en ce que** l'huile de silicone est choisie parmi les polydiméthylsiloxanes, les polyméthylphénylsiloxanes, les silicones comportant des séquences ou des greffons polyoxyalkylènes, en particulier polyoxyéthylène ou copoly(oxyéthylène/oxypropylène) telles que les diméthiconecopolyols, les silicones portant à la fois des groupes hydrophobes hydrocarbonés (par exemple des groupes alkyle en C₂-C₃₀) et des séquences ou greffons polyoxyéthylénés ou copoly(oxyéthylénés/oxypropylénés) telles que les alkyldiméthiconecopolyols, les silicones portant des groupes fluorés ou perfluorés telles que les polydiméthylsiloxanes perfluoroalkylés et les polyméthylphénylsiloxanes perfluoroalkylés.

14. Procédé selon l'une quelconque des revendications précédentes, **caractérisé en ce qu'**il permet d'accroître la persistance de la coloration et/ou de la brillance d'un rouge à lèvres, d'un mascara ou d'un eye-liner.

15. Procédé selon l'une quelconque des revendications précédentes, **caractérisé en ce qu'**il permet d'accroître la persistance de la matité et/ou de la coloration des fonds de teint, des poudres, des fards à joues, des ombres à paupières ou des maquillages de corps.

16. Procédé selon l'une quelconque des revendications précédcntes, **caractérisé en ce qu'**il permet d'accroître la persistance d'un actif de soin pour des produits hydratants, des déodorants ou des anti-transpirants.

17. Composition cosmétique comprenant une phase huileuse qui comporte au moins une huile de silicone, volatile ou non, à laquelle on a ajouté une quantité efficace d'au moins un organopolysiloxane, linéaire ou cyclique, comportant au moins deux motifs organosiloxy et au moins deux groupes latéraux et/ou terminaux capables d'établir chacun au moins une liaison hydrogène avec un ou des groupes partenaires, **caractérisée en ce que** lesdits motifs organosiloxy sont représentés par la formule suivante :
RₐR'_{b}SiO_{(4-a-b)/2}
dans laquelle :
R représente un groupe alkyle linéaire, ramifié ou cyclique, un groupe aryle, un groupe polyéther ou un groupe fluoré,
R' représente un groupe capable d'établir au moins une liaison hydrogène,
a vaut 1, 2 ou 3, et
b vaut 0 ou 1, à condition que a+b soit égal à 2 ou 3,
ledit groupe R' étant choisi parmi :
(a) les groupes dérivés d'acides aminés non protégés ou partiellement protégés, et
(b) les groupes acides carboxyliques, amines ou phénols de formule :
-X-(Y)ₙ-Z
dans laquelle :
X représente une chaîne d'espacement linéaire, ramifiée ou cyclique, de type alkylène ou alcénylène, comportant éventuellement un ou plusieurs hétéroatomes dans la chaîne ;
Y représente un groupe hydrocarboné insaturé, divalent, monocyclique ou polycyclique, ou un groupe hétérocyclique insaturé divalent, ces groupes polycycliques ou hétérocycliques pouvant comporter jusqu'à 4 cycles condensés,
n représente un nombre entier allant de 1 à 4, et
Z représente un groupe -COOH, -OH, ou un groupe amine primaire, secondaire ou tertiaire, dont l'atome d'azote fait éventuellement partie d'un groupe hétérocyclique Y.

18. Composition cosmétique selon la revendication 17, **caractérisée en ce que** l'organopolysiloxane comporte de 2 à 50 000 motifs organosiloxy, de préférence de 2 à 30 000 motifs organosiloxy.

19. Composition cosmétique selon la revendication 17 ou 18, **caractérisée en ce que** les groupes latéraux et/ou terminaux sont capables d'établir chacun au moins deux liaisons hydrogène avec un ou des groupes partenaires.

20. Composition cosmétique selon l'une quelconque des revendications 17 à 19, **caractérisée en ce que** Y représente un noyau aromatique à 6 chaînons et Z représente un groupe -COOH.

21. Composition cosmétique selon la revendication 20, **caractérisée en ce que** l'organopolysiloxane est représenté par la formule suivante : 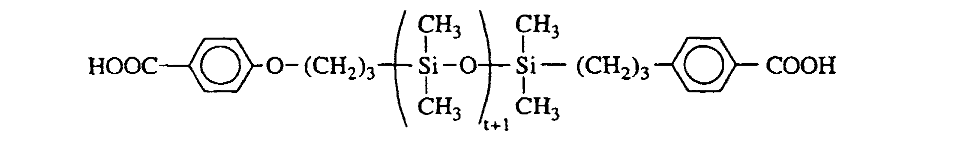 avec t allant de préférence de 1 à 1200.

22. Composition cosmétique selon la revendication 21, **caractérisée en ce que** l'organopolysiloxane est représenté par la formule suivante :

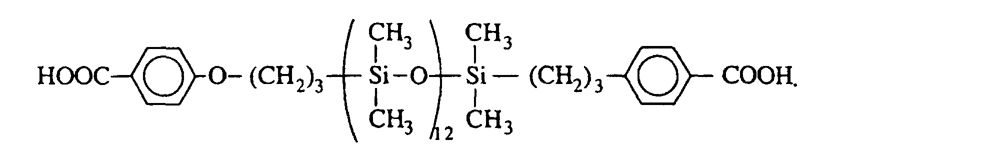

23. Composition cosmétique selon l'une quelconque des revendications 17 à 19, **caractérisée en ce que** lorsque Z représente un groupe amino, l'atome d'azote fait partie d'un groupe hétérocyclique Y, et Y-Z représente un groupe pyridyle.

24. Composition cosmétique selon l'une quelconque des revendications 17 à 23, **caractérisée en ce que** la quantité efficace de l'organo-polysiloxane est comprise entre 0,5 et 50 % en poids, par rapport au poids total de la composition cosmétique.

25. Composition cosmétique selon la revendication 24, **caractérisée en ce que** la quantité efficace de l'organopolysiloxane est comprise entre 1 et 30 % en poids, par rapport au poids total de la composition cosmétique.

26. Composition cosmétique selon l'une quelconque des revendications 17 à 25, **caractérisée en ce que** la phase huileuse comprend en outre au moins une huile hydrocarbonée.

27. Composition cosmétique selon l'une quelconque des revendications 17 à 26, **caractérisée en ce que** l'huile de silicone est choisie parmi les polydiméthylsiloxanes (PDMS), éventuellement phénylés tels que les phényltriméthicones, les phényltriméthylsiloxydiphénylsiloxanes, les diphénylméthyldiméthyltrisiloxanes, les diphényldiméthicones, les phényldiméthicones, les polyméthylphénylsiloxanes, éventuellement substitués par des groupements aliphatiques et/ou aromatiques, ou éventuellement fluorés ; les polysiloxanes modifiés par des acides gras, des alcools gras ou des polyoxyalkylènes, les silicones fluorées et les huiles siliconées perfluorées.

28. Composition cosmétique selon la revendication 27, **caractérisée en ce que** l'huile de silicone est choisie parmi les polydiméthylsiloxanes, les polyméthylphénylsiloxanes, les silicones comportant des séquences ou des greffons polyoxyalkylènes, en particulier polyoxyéthylène ou copoly(oxyéthylène/oxypropylène) telles que les diméthiconecopolyols, les silicones portant à la fois des groupes hydrophobes hydrocarbonés (par exemple des groupes alkyle en C₂-C₃₀) et des séquences ou greffons polyoxyéthylénés ou copoly(oxyéthylénés/oxypropylénés) telles que les alkyldiméthiconecopolyols, les silicones portant des groupes fluorés ou perfluorés telles que les polydiméthylsiloxanes perfluoroalkylés et les polyméthylphénylsiloxanes perfluoroalkylés.

29. Composition cosmétique selon l'une quelconque des revendications 17 à 28, **caractérisée en ce qu'**elle comprend en outre des additifs choisis parmi les charges, les pigments, les colorants, les tensio-actifs, les filtres solaires, les cires naturelles ou synthétiques, les anti-oxydants, les parfums et les conservateurs.

30. Composition cosmétique selon l'une quelconque des revendications 17 à 29, **caractérisée en ce qu'**elle est anhydre.

31. Composition cosmétique selon l'une quelconque des revendications 17 à 30, **caractérisée en ce qu'**elle se présente sous la forme d'un stick ou d'un bâton, sous la forme d'une pâte souple, de viscosité dynamique à 25 °C de l'ordre de 1 à 40 Pa.s, sous forme de coupelle, de gel huileux ou de liquide huileux.

32. Composition cosmétique selon l'une quelconque des revendications 17 à 31, **caractérisée en ce qu'**elle est utilisée pour le maquillage et/ou le soin de la peau, y compris celle des paupières, ainsi que des lèvres et des phanères.

33. Composition cosmétique selon l'une quelconque des revendications 17 à 32, **caractérisée en ce qu'**elle se présente sous la forme d'un rouge à lèvres, d'un mascara, d'un eye-liner, d'un fond de teint, d'une poudre, d'un fard à joues, d'ombre à paupières ou d'un maquillage de corps.

34. Composition cosmétique selon l'une quelconque des revendications 17 à 32, **caractérisée en ce qu'**elle se présente sous la forme d'un produit hydratant, d'un déodorant ou d'un anti-transpirant.

35. Utilisation d'une composition cosmétique selon l'une quelconque des revendications 17 à 32, pour accroître la persistance de la coloration et/ou la brillance d'un rouge à lèvres, d'un mascara, d'un eye-liner.

36. Utilisation d'une composition cosmétique selon l'une quelconque des revendications 17 à 32, pour accroître la persistance de la matité et/ou de la coloration d'un fond de teint, d'une poudre, d'un fard à joues, d'une ombre à paupières ou d'un maquillage de corps.

37. Utilisation d'une composition cosmétique selon l'une quelconque des revendications 17 à 32, pour accroître la persistance de l'effet de soin d'un produit hydratant.

38. Utilisation d'une composition cosmétique selon l'une quelconque des revendications 17 à 32, pour supprimer durablement les odeurs corporelles, dans un déodorant ou un anti-transpirant.

## Claims

1. Process for increasing the persistence of at least one cosmetic and/or care effect of a cosmetic composition comprising an oily phase, **characterized in that** it comprises the incorporation into the composition of an effective amount of at least one linear or cyclic polyorganosiloxane comprising at least two organosiloxy units and at least two side and/or end groups each capable of forming at least one hydrogen bond with one or more partner groups, the said organosiloxy units being represented by the following formula:
Rₐ R'_{b}SiO_{(4-a-b)/2}
in which:
R represents a linear, branched or cyclic alkyl group, an aryl group, a polyether group or a fluoro group,
R' represents a group capable of forming at least one hydrogen bond,
a is 1, 2 or 3, and
b is 0 or 1, with the proviso that a+b is equal to 2 or 3;
and the said groups R' capable of forming at least one hydrogen bond being chosen from:
(a) groups derived from unprotected or partially protected amino acids, and
(b) carboxylic acid, amine or phenol groups of formula:
-X- (Y)ₙ-Z
in which:
X represents a linear, branched or cyclic spacer chain, of alkylene or alkenylene type, optionally comprising one or more hetero atoms in the chain,
Y represents a monocyclic or polycyclic divalent unsaturated hydrocarbon-based group or a divalent unsaturated heterocyclic group, these polycyclic or heterocyclic groups possibly comprising up to 4 fused rings,
n represents an integer ranging from 1 to 4, and
Z represents a -COOH or -OH group or a primary, secondary or tertiary amine group, the nitrogen atom of which optionally forms part of a heterocyclic group Y.

2. Process according to Claim 1, **characterized in that** the polyorganosiloxane comprises from 2 to 50,000 organosiloxy units and preferably from 2 to 30,000 organosiloxy units.

3. Process according to Claim 1 or 2, **characterized in that** the side and/or end groups are each capable of forming at least two hydrogen bonds with one or more partner groups.

4. Process according to any one of the preceding claims, **characterized in that** Y represents a 6-membered aromatic nucleus and Z represents a -COOH group.

5. Process according to any one of the preceding claims, **characterized in that** the polyorganosiloxane is represented by the following formula: 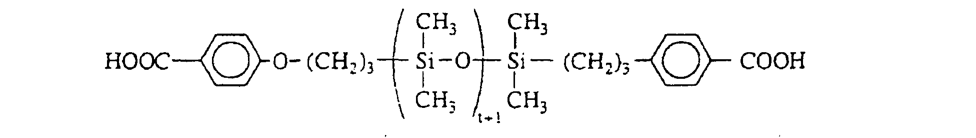 with t preferably ranging from 1 to 1200.

6. Process according to Claim 5, **characterized in that** the polyorganosiloxane is represented by the following formula:

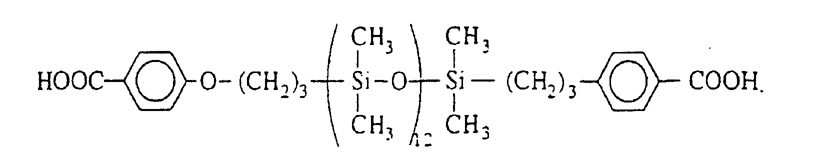

7. Process according to any one of Claims 1 to 3, **characterized in that**, when Z represents an amine group, the nitrogen atom forms part of a heterocyclic group Y, and Y-Z represents a pyridyl group.

8. Process according to any one of the preceding claims, **characterized in that** the effective amount of the polyorganosiloxane is between 0.5% and 50% by weight relative to the total weight of the cosmetic composition.

9. Process according to Claim 8, **characterized in that** the effective amount of the polyorganosiloxane is between 1% and 30% by weight relative to the total weight of the cosmetic composition.

10. Process according to any one of the preceding claims, **characterized in that** the oily phase comprises at least one hydrocarbon-based oil and/or at least one silicone oil.

11. Process according to Claim 10, **characterized in that** the oily phase contains at least one volatile or non-volatile silicone oil.

12. Process according to Claim 10 or 11, **characterized in that** the silicone oil is chosen from polydimethylsiloxanes (PDMSs), that are optionally phenylated, such as phenyltrimethicones, phenyltrimethylsiloxydiphenylsiloxanes, diphenylmethyldimethyltrisiloxanes, diphenyldimethicones, phenyldimethicones and polymethylphenylsiloxanes, optionally substituted with aliphatic and/or aromatic groups, or optionally fluorinated; polysiloxanes modified with fatty acids, fatty alcohols or polyoxyalkylenes, fluorosilicones and perfluorosilicone oils.

13. Process according to Claim 12, **characterized in that** the silicone oil is chosen from polydimethylsiloxanes, polymethylphenylsiloxanes, silicones comprising polyoxyalkylene blocks or grafts, in particular polyoxyethylene or copoly(oxyethylene/oxypropylene) blocks or grafts, such as dimethicone copolyols, silicones bearing both hydrophobic hydrocarbon-based groups (for example C₂-C₃₀ alkyl groups) and polyoxyethylenated or copoly(oxyethylenated/oxypropylenated) blocks or grafts, such as alkyldimethicone copolyols, silicones bearing fluoro or perfluoro groups such as perfluoroalkyl polydimethylsiloxanes and perfluoroalkyl polymethylphenylsiloxanes.

14. Process according to any one of the preceding claims, **characterized in that** it increases the persistence of the colouring effect and/or gloss of a lipstick, a mascara or an eyeliner.

15. Process according to any one of the preceding claims, **characterized in that** it increases the persistence of the matt-effect and/or colouring effect of foundations, powders, blushers, eyeshadows or body make-up.

16. Process according to any one of the preceding claims, **characterized in that** it increases the persistence of an active care agent for moisturizing products, deodorants or antiperspirants.

17. Cosmetic composition comprising an oily phase which comprises at least one volatile or non-volatile silicone oil, to which is added an effective amount of at least one linear or cyclic polyorganosiloxane comprising at least two organosiloxy units and at least two side and/or end groups which are each capable of forming at least one hydrogen bond with one or more partner groups, **characterized in that** the said organosiloxy units are represented by the following formula:
Rₐ R'_{b}SiO_{(4-a-b)/2}
in which:
R represents a linear, branched or cyclic alkyl group, an aryl group, a polyether group or a fluoro group,
R' represents a group capable of forming at least one hydrogen bond,
a is 1, 2 or 3, and
b is 0 or 1, with the proviso that a+b is equal to 2 or 3,
the said group R' being chosen from:
(a) groups derived from unprotected or partially protected amino acids, and
(b) carboxylic acid, amine or phenol groups of formula:
-X- (Y) ₙ-Z
in which:
X represents a linear, branched or cyclic spacer chain, of alkylene or alkenylene type, optionally comprising one or more hetero atoms in the chain,
Y represents a monocyclic or polycyclic divalent unsaturated hydrocarbon-based group or a divalent unsaturated heterocyclic group, these polycyclic or heterocyclic groups possibly comprising up to 4 fused rings,
n represents an integer ranging from 1 to 4, and
Z represents a -COOH or -OH group or a primary, secondary or tertiary amine group, the nitrogen atom of which optionally forms part of the heterocyclic group Y.

18. Cosmetic composition according to Claim 17, **characterized in that** the polyorganosiloxane comprises from 2 to 50,000 organosiloxy units and preferably from 2 to 30,000 organosiloxy units.

19. Cosmetic composition according to Claim 17 or 18, **characterized in that** the side and/or end groups are each capable of forming at least two hydrogen bonds with one or more partner groups.

20. Cosmetic composition according to any one of Claims 17 to 19, **characterized in that** Y represents a 6-membered aromatic nucleus and Z represents a -COOH group.

21. Cosmetic composition according to Claim 20, **characterized in that** the polyorganosiloxane is represented by the following formula: 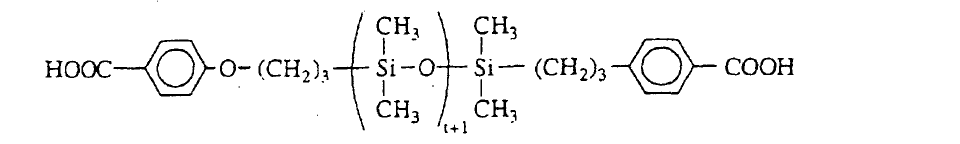 with t preferably ranging from 1 to 1200.

22. Cosmetic composition according to Claim 21, **characterized in that** the polyorganosiloxane is represented by the following formula:

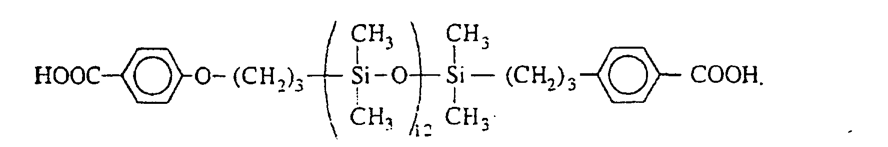

23. Cosmetic composition according to any one of Claims 17 to 19, **characterized in that**, when Z represents an amine group, the nitrogen atom forms part of a heterocyclic group Y, and Y-Z represents a pyridyl group.

24. Cosmetic composition according to any one of Claims 17 to 23, **characterized in that** the effective amount of the polyorganosiloxane is between 0.5% and 50% by weight relative to the total weight of the cosmetic composition.

25. Cosmetic composition according to Claim 24, **characterized in that** the effective amount of the polyorganosiloxane is between 1% and 30% by weight relative to the total weight of the cosmetic composition.

26. Cosmetic composition according to any one of Claims 17 to 25, **characterized in that** the oily phase also comprises at least one hydrocarbon-based oil.

27. Cosmetic composition according to any one of Claims 17 to 26, **characterized in that** the silicone oil is chosen from polydimethylsiloxanes (PDMSs), that are optionally phenylated, such as phenyltrimethicones, phenyltrimethylsiloxydiphenylsiloxanes, diphenylmethyldimethyltrisiloxanes, diphenyldimethicones, phenyldimethicones and polymethylphenylsiloxanes, optionally substituted with aliphatic and/or aromatic groups, or optionally fluorinated; polysiloxanes modified with fatty acids, fatty alcohols or polyoxyalkylenes, fluorosilicones and perfluorosilicone oils.

28. Cosmetic composition according to Claim 27, **characterized in that** the silicone oil is chosen from polydimethylsiloxanes, polymethylphenylsiloxanes, silicones comprising polyoxyalkylene blocks or grafts, in particular polyoxyethylene or copoly(oxyethylene/oxypropylene) blocks or grafts, such as dimethicone copolyols, silicones bearing both hydrophobic hydrocarbon-based groups (for example C₂-C₃₀ alkyl groups) and polyoxyethylenated or copoly(oxyethylenated/oxypropylenated) blocks or grafts, such as alkyldimethicone copolyols, silicones bearing fluoro or perfluoro groups such as perfluoroalkyl polydimethylsiloxanes and perfluoroalkyl polymethylphenylsiloxanes.

29. Cosmetic composition according to any one of Claims 17 to 28, **characterized in that** it also comprises additives chosen from fillers, pigments, colourants, surfactants, sunscreens, natural or synthetic waxes, antioxidants, fragrances and preserving agents.

30. Cosmetic composition according to any one of Claims 17 to 29, **characterized in that** it is anhydrous.

31. Cosmetic composition according to any one of Claims 14 to 30, **characterized in that** it is in the form of a stick or tube, in the form of a soft paste, with a dynamic viscosity at 25°C of about from 1 to 40 Pa.s, or in the form of a dish, an oily gel or an oily liquid.

32. Cosmetic composition according to any one of Claims 17 to 31, **characterized in that** it is used for making up and/or caring for the skin, including the skin of the eyelids, and also the lips and superficial body growths.

33. Cosmetic composition according to any one of Claims 17 to 32, **characterized in that** it is in the form of a lipstick, a mascara, an eyeliner, a foundation, a powder, a blusher, an eyeshadow or a body make-up.

34. Cosmetic composition according to any one of Claims 17 to 32, **characterized in that** it is in the form of a moisturizing product, a deodorant or an antiperspirant.

35. Use of a cosmetic composition according to any one of Claims 17 to 32, to increase the persistence of the colouring effect and/or gloss of a lipstick, a mascara or an eyeliner.

36. Use of a cosmetic composition according to any one of Claims 17 to 32, to increase the persistence of the matt effect and/or colouring effect of a foundation, a powder, a blusher, an eyeshadow or a body make-up.

37. Use of a cosmetic composition according to any one of Claims 17 to 32, to increase the persistence of the care effect of a moisturizing product.

38. Use of a cosmetic composition according to any one of Claims 17 to 32, to durably eliminate body odours, in a deodorant or an antiperspirant.

## Patentansprüche

1. Verfahren zur Verlängerung der Dauer mindestens eines kosmetischen Effekts und/oder einer pflegenden Wirkung einer kosmetischen Zusammensetzung, die eine Ölphase enthält, **dadurch gekennzeichnet, dass** es umfasst, mindestens ein geradkettiges oder cyclisches Organopolysiloxan, das mindestens zwei Organosiloxyeinheiten und mindestens zwei Seitengruppen und/oder endständige Gruppen aufweist, die befähigt sind, jeweils mindestens eine Wasserstoff-Brückenbindung mit einem oder mehreren Bindungspartnern auszubilden, in einer wirksamen Menge in die Zusammensetzung einzuarbeiten, wobei die Organosiloxyeinheiten durch die folgende Formel dargestellt werden können:
RₐR'_{b}SiO_{(4-a-b)/2}
worin bedeuten:
R eine geradkettige, verzweigte oder cyclische Alkylgruppe, eine Arylgruppe, eine Polyethergruppe oder eine fluorierte Gruppe,
R' eine Gruppe, die befähigt ist, mindestens eine Wasserstoff-Brückenbindung auszubilden,
a 1, 2 oder 3, und
b 0 oder 1, mit der Maßgabe, dass a + b 2 oder 3 bedeutet, wobei die Gruppe R' ausgewählt ist unter:
(a) den Gruppen, die von nicht geschützten oder teilweise geschützten Aminosäuren abgeleitet sind, und
(b) den Carbonsäuregruppen, Aminogruppen oder Phenolgruppen der Formel:
-X-(Y)ₙ-Z
worin bedeuten:
X eine geradkettige, verzweigte oder cyclische Verbindungsgruppe vom Alkylentyp oder Alkenylentyp, die in der Kette gegebenenfalls ein oder mehrer Heteroatome enthält;
Y eine ungesättigte, zweiwertige, monocyclische oder polycyclische Kohlenwasserstoffgruppe oder eine zweiwertige ungesättigte heterocyclische Gruppe, wobei die polycyclischen oder heterocyclischen Gruppen bis zu vier kondensierte Ringe aufweisen können,
n eine ganze Zahl von 1 bis 4, und
Z die Gruppe -COOH, die Gruppe -OH oder eine primäre, sekundäre oder tertiäre Aminogruppe, deren Stickstoffatom gegebenenfalls Teil einer heterocyclischen Gruppe Y ist.

2. Verfahren nach Anspruch 1, **dadurch gekennzeichnet, dass** das Organopolysiloxan 2 bis 50 000 Organosiloxyeinheiten und vorzugsweise 2 bis 30 000 Organosiloxyeinheiten aufweist.

3. Verfahren nach Anspruch 1 oder 2, **dadurch gekennzeichnet, dass** die Seitengruppen und/oder endständigen Gruppen befähigt sind, jeweils mindestens zwei Wasserstoff-Brückenbindungen mit einem oder mehreren Bindungspartnern einzugehen.

4. Verfahren nach einem der vorhergehenden Ansprüche, **dadurch gekennzeichnet, dass** Y ein sechsgliedriger aromatischer Ring ist und Z die Gruppe -COOH bedeutet.

5. Verfahren nach einem der vorhergehenden Ansprüche, **dadurch gekennzeichnet, dass** das Organopolysiloxan der folgenden Formel entspricht: 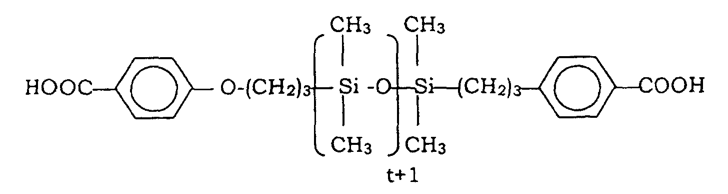 wobei t im Bereich von 1 bis 1200 liegt.

6. Verfahren nach Anspruch 5, **dadurch gekennzeichnet, dass** das Organopolysiloxan der folgenden Formel entspricht:

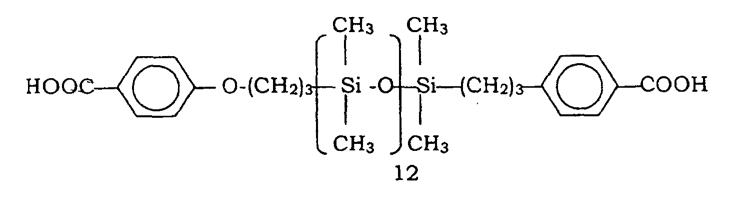

7. Verfahren nach einem der Ansprüche 1 bis 3, **dadurch gekennzeichnet, dass**, wenn Z eine Aminogruppe bedeutet, das Stickstoffatom Teil einer heterocyclischen Gruppe Y ist, wobei Y-Z eine Pyridylgruppe bedeutet.

8. Verfahren nach einem der vorhergehenden Ansprüche, **dadurch gekennzeichnet, dass** die wirksame Menge des Organopolysiloxan im Bereich von 0,5 bis 50 Gew.-%, bezogen auf das Gesamtgewicht der Zusammensetzung, liegt.

9. Verfahren nach Anspruch 8, **dadurch gekennzeichnet, dass** die wirksame Menge des Organopolysiloxan im Bereich von 1 bis 30 Gew.-%, bezogen auf das Gesamtgewicht der kosmetischen Zusammensetzung, liegt.

10. Verfahren nach einem der vorhergehenden Ansprüche, **dadurch gekennzeichnet, dass** die Ölphase mindestens ein Kohlenwasserstofföl und/ oder mindestens ein Siliconöl enthält.

11. Verfahren nach Anspruch 10, **dadurch gekennzeichnet, dass** die Ölphase mindestens ein flüchtiges oder nicht flüchtiges Siliconöl enthält.

12. Verfahren nach Anspruch 10 oder 11, **dadurch gekennzeichnet, dass** das Siliconöl unter den Polydimethylsiloxanen (PDMS), die gegebenenfalls phenyliert sind, wie beispielsweise Phenyltrimethiconen, Phenyltrimethylsiloxydiphenylsiloxanen, Diphenylmethyldimethyltrisiloxanen, Diphenyldimethiconen, Phenyldimethiconen, Polymethylphenylsiloxanen, die gegebenenfalls mit aliphatischen und/oder aromatischen Gruppen substituiert oder gegebenenfalls fluoriert sind; den Polysiloxanen, die mit Fettsäuren, Fettalkoholen oder Polyoxyalkylenen modifiziert sind, den fluorierten Siliconen und den perfluorierten Siliconölen ausgewählt ist.

13. Verfahren nach Anspruch 12, **dadurch gekennzeichnet, dass** das Siliconöl unter den Polydimethylsiloxanen, Polymethylphenylsiloxanen, Siliconen, die Polyoxyalkylensequenzen oder Polyoxyalkylenpfropfzweige enthalten, insbesondere Sequenzen oder Pfropfzweige von Polyethylenoxid oder Copoly(ethylenoxid/propylenoxid), wie Dimethiconcopolyolen, Siliconen, die gleichzeitig hydrophobe Kohlenwasserstoffgruppen (beispielsweise C₂₋₃₀-Alkylgruppen) und polyethoxylierte oder copoly(ethoxylierte/propoxylierte) Sequenzen oder Pfropfzweige enthalten, wie Alkyldimethiconcopolyolen, Siliconen, die fluorierte oder perfluorierte Gruppen enthalten, wie perfluoralkylierten Polydimethylsiloxanen und perfluoralkylierten Polymethylphenylsiloxanen, ausgewählt ist.

14. Verfahren nach einem der vorhergehenden Ansprüche, **dadurch gekennzeichnet, dass** es ermöglicht, die Dauerhaftigkeit der Farbe und/oder des Glanzes von Lippenstiften, Mascaras oder Eyelinern zu verlängern.

15. Verfahren nach einem der vorhergehenden Ansprüche, **dadurch gekennzeichnet, dass** es ermöglicht, das Anhalten der Mattigkeit und/oder der Farbe von Make-up, Pudern, Wangenrouge, Lidschatten oder Schminke für den Körper zu verbessern.

16. Verfahren nach einem der vorhergehenden Ansprüche, **dadurch gekennzeichnet, dass** es ermöglicht, die Dauer der pflegenden Wirkung von hydratisierenden Produkten, Deodorants oder Antitranspirantien zu verlängern.

17. Kosmetische Zusammensetzung, die eine Ölphase enthält, die mindestens ein flüchtiges oder nicht flüchtiges Siliconöl umfasst und in die in einer wirksamen Menge mindestens ein geradkettiges oder cyclisches Organopolysiloxan eingearbeitet wurde, das mindestens zwei Organosiloxyeinheiten und mindestens zwei Seitengruppen und/oder endständige Gruppen aufweist, die befähigt sind, jeweils mindestens eine Wasserstoff-Brückenbindung mit einem oder mehreren Bindungspartnern einzugehen, **dadurch gekennzeichnet, dass** die Organosiloxyeinheiten der folgenden Formel entsprechen:
RₐR'_{b}SiO_{(4-a-b)/2}
worin bedeuten:
R eine geradkettige, verzweigte oder cyclische Alkylgruppe, eine Arylgruppe, eine Polyethergruppe oder eine fluorierte Gruppe, R' eine Gruppe, die befähigt ist, mindestens eine Wasserstoff-Brückenbindung auszubilden,
a 1, 2 oder 3, und
b 0 oder 1, mit der Maßgabe, dass a + b 2 oder 3 bedeutet, wobei die Gruppe R' ausgewählt ist unter:
(c) den Gruppen, die von nicht geschützten oder teilweise geschützten Aminosäuren abgeleitet sind, und
(d) den Carbonsäuregruppen, Aminogruppen oder Phenolgruppen der Formel:
-X-(Y)ₙ-Z
worin bedeuten:
X eine geradkettige, verzweigte oder cyclische Verbindungsgruppe vom Alkylentyp oder Alkenylentyp, die in der Kette gegebenenfalls ein oder mehrer Heteroatome enthält;
Y eine ungesättigte, zweiwertige, monocyclische oder polycyclische Kohlenwasserstoffgruppe oder eine zweiwertige ungesättigte heterocyclische Gruppe, wobei die polycyclischen oder heterocyclischen Gruppen bis zu 4 kondensierte Ringe aufweisen können,
n eine ganze Zahl von 1 bis 4, und
Z die Gruppe -COOH, die Gruppe -OH oder eine primäre, sekundäre oder tertiäre Aminogruppe, deren Stickstoffatom gegebenenfalls Teil einer heterocyclischen Gruppe Y ist.

18. Kosmetische Zusammensetzung nach Anspruch 17, **dadurch gekennzeichnet, dass** das Organopolysiloxan 2 bis 50 000 Organosiloxyeinheiten und vorzugsweise 2 bis 30 000 Organosiloxyeinheiten aufweist.

19. Kosmetische Zusammensetzung nach Anspruch 17 oder 18, **dadurch gekennzeichnet, dass** die Seitengruppen und/oder endständigen Gruppen befähigt sind, jeweils mindestens zwei Wasserstoff-Brückenbindungen mit einem oder mehreren Bindungspartnern auszubilden.

20. Kosmetische Zusammensetzung nach einem der Ansprüche 17 bis 19, **dadurch gekennzeichnet, dass** Y einen sechsgliedrigen aromatischen Ring und Z die Gruppe -COOH bedeutet.

21. Kosmetische Zusammensetzung nach Anspruch 20, **dadurch gekennzeichnet, dass** das Organopolysiloxan der folgenden Formel entspricht: 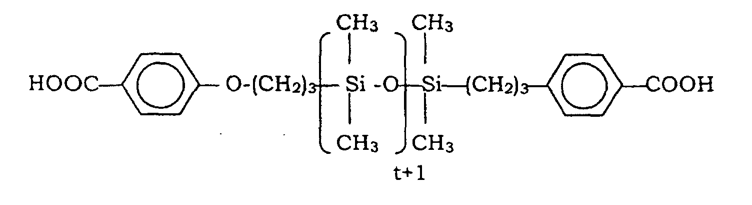 wobei t im Bereich von 1 bis 1200 liegt.

22. Kosmetische Zusammensetzung nach Anspruch 21, **dadurch gekennzeichnet, dass** das Organopolysiloxan der folgenden Formel entspricht:

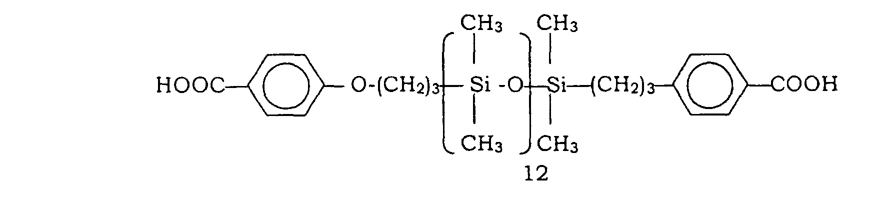

23. Kosmetische Zusammensetzung nach einem der Ansprüche 17 bis 19, **dadurch gekennzeichnet, dass**, wenn Z eine Aminogruppe bedeutet, das Stickstoffatom Teil einer heterocyclischen Gruppe Y ist, wobei Y-Z eine Pyridylgruppe bedeutet.

24. Kosmetische Zusammensetzung nach einem der Ansprüche 17 bis 23, **dadurch gekennzeichnet, dass** die wirksame Menge des Organopolysiloxan im Bereich von 0,5 bis 50 Gew.-%, bezogen auf das Gesamtgewicht der kosmetischen Zusammensetzung, liegt.

25. Kosmetische Zusammensetzung nach Anspruch 24, **dadurch gekennzeichnet, dass** die wirksame Menge des Organopolysiloxan im Bereich von 1 bis 30 Gew.-%, bezogen auf das Gesamtgewicht der kosmetischen Zusammensetzung, liegt.

26. Kosmetische Zusammensetzung nach einem der Ansprüche 17 bis 25, **dadurch gekennzeichnet, dass** die Ölphase ferner mindestens ein Kohlenwasserstofföl enthält.

27. Kosmetische Zusammensetzung nach einem der Ansprüche 17 bis 26, **dadurch gekennzeichnet, dass** das Siliconöl unter den Polydimethylsiloxanen (PDMS), die gegebenenfalls phenyliert sind, wie beispielsweise Phenyltrimethiconen, Phenyltrimethylsiloxydiphenylsiloxanen, Diphenylmethyldimethyltrisiloxanen, Diphenyldimethiconen, Phenyldimethiconen, Polymethylphenylsiloxanen, die gegebenenfalls mit aliphatischen und/oder aromatischen Gruppen substituiert oder gegebenenfalls fluoriert sind; den Polysiloxanen, die mit Fettsäuren, Fettalkoholen oder Polyalkylenoxiden modifiziert sind, den fluorierten Siliconen und den perfluorierten Siliconölen ausgewählt ist.

28. Kosmetische Zusammensetzung nach Anspruch 27, **dadurch gekennzeichnet, dass** das Siliconöl unter den Polydimethylsiloxanen, Polymethylphenylsiloxanen, Siliconen, die Polyoxyalkylensequenzen oder Polyoxyalkylenpfropfzweige enthalten, insbesondere Sequenzen oder Pfropfzweige von Polyethylenoxid oder Copoly(ethylenoxid/propylenoxid), wie Dimethiconcopolyolen, Siliconen, die gleichzeitig hydrophobe Kohlenwasserstoffgruppen (beispielsweise C₂₋₃₀-Alkylgruppen) und polyethoxylierte oder copoly(ethoxylierte/propoxylierte) Sequenzen oder Pfropfzweige enthalten, wie Alkyldimethiconcopolyolen, Siliconen, die fluorierte oder perfluorierte Gruppen enthalten, wie perfluoralkylierten Polydimethylsiloxanen und perfluoralkylierten Polymethylphenylsiloxanen, ausgewählt ist.

29. Kosmetische Zusammensetzung nach einem der Ansprüche 17 bis 28, **dadurch gekennzeichnet, dass** sie ferner Zusatzstoffe enthält, die unter den Füllstoffen, Pigmenten, Farbstoffen, grenzflächenaktiven Stoffen, Sonnenschutzfiltern, natürlichen oder synthetischen Wachsen, Antioxidantien, Parfums und Konservierungsmitteln ausgewählt sind.

30. Kosmetische Zusammensetzung nach einem der Ansprüche 17 bis 29, **dadurch gekennzeichnet, dass** sie wasserfrei ist.

31. Kosmetische Zusammensetzung nach einem der Ansprüche 17 bis 30, **dadurch gekennzeichnet, dass** sie als Stift oder Stick, in Form einer weichen Paste mit einer dynamischen Viskosität in der Größenordnung von 1 bis 40 Pa·s bei 25° C, in Tiegelform oder als öliges Gel oder ölige Flüssigkeit vorliegt.

32. Kosmetische Zusammensetzung nach einem der Ansprüche 17 bis 31, **dadurch gekennzeichnet, dass** sie zum Schminken und/oder zur Pflege der Haut einschließlich der Lider sowie der Lippen und der Hautanhangsgebilde verwendet wird.

33. Kosmetische Zusammensetzung nach einem der Ansprüche 17 bis 32, **dadurch gekennzeichnet, dass** sie als Lippenstift, Mascara, Eyeliner, Make-up, Puder, Wangenrouge, Lidschatten oder Zusammensetzung zum Schminken des Körpers vorliegt.

34. Kosmetische Zusammensetzung nach einem der Ansprüche 17 bis 32, **dadurch gekennzeichnet, dass** sie in Form eines hydratisierenden Produktes, als Deodorant oder als Antitranspirant vorliegt.

35. Verwendung einer kosmetischen Zusammensetzung nach einem der Ansprüche 17 bis 32 zur Verbesserung der Dauerhaftigkeit der Farbe und/oder des Glanzes von Lippenstiften, Mascaras oder Eyelinern.

36. Verwendung einer kosmetischen Zusammensetzung nach einem der Ansprüche 17 bis 32 zur Verbesserung der Dauerhaftigkeit der Mattigkeit und/oder der Farbe von Make-up, Pudern, Wangenrouge, Lidschatten oder Schminke für den Körper.

37. Verwendung einer kosmetischen Zusammensetzung nach einem der Ansprüche 17 bis 32 zur Verbesserung der Dauerhaftigkeit der pflegenden Wirkung von hydratisierenden Produkten.

38. Verwendung einer kosmetischen Zusammensetzung nach einem der Ansprüche 17 bis 32, um im Falle von Deodorants oder Antitranspirantien Körpergerüche dauerhaft zu unterdrücken.
